# EUROPÄISCHE PATENTANMELDUNG

(11) **EP 1 752 126 A1**
(43) Veröffentlichungstag der Anmeldung: **14.02.2007**
(21) Anmeldenummer: 05017586.8
(22) Anmeldetag: 12.08.2005
(51) Int. Cl.: A61H 35/00

(54) **Elektrolysefussbadsystem**

(71) Anmelder: Body Detox AG, 8400 Winterthur (CH)
(72) Erfinder: Mosimann, François, 8400 Winterthur (CH); Artmann, Heribert, 93455 Traitsching - Höhhof (DE)
(74) Vertreter: Koepe, Gerd L.

(57) **Zusammenfassung**

Ein Elektrolysefußbadsystem weist eine Fußbadwanne und einen Fußbadständer auf, auf dem die Fußbadwanne abgestützt ist.

## Beschreibung

Die Erfindung betrifft ein Elektrolysefußbadsystem.

In der Medizin, in der Heilkunde und im Wellness-Bereich werden Elektrolysefußbäder als therapeutische Maßnahme zur Entschlackung und Entgiftung des menschlichen Körpers verordnet. Hierbei werden über die Haut der in eine Solelösung eingetauchten Füße toxische oder belastende aber auch körpereigene Stoffe ausgeschieden. Insbesondere werden bei der Anwendung des Elektrolysefußbads Schwermetalle, Harnsäure, Calcium, Magnesium, chlorierte Kohlenwasserstoffe, Biphenyle, usw. aus dem Körper ausgeleitet.

Zur Durchführung eines Elektrolysefußbads werden herkömmliche Elektrolysefußbadgeräte verwendet, die eine Wanne und eine Konverterspule aufweisen. Die Wanne ist mit eventuell mit Zusätzen versehenem Wasser gefüllt, in das die Konverterspule eingetaucht ist. Die Konverterspule weist zwei Elektroden auf, die zueinander ein elektrisches Potential haben, aufgrund dessen eine Ionisierung des Wassers erzeugt wird. Sobald ein Patient seine Füße in das Wasser eintaucht, tritt über die von der Konverterspule erzeugte Ionisierung des Wassers die Wirkung des Elektrolysefußbads ein.

Beim Fußbaden sitzt der Patient beispielsweise auf einem Stuhl vor der Wanne. Zu Beginn des Fußbads stellt der Patient seine Füße von außerhalb der Wanne in die Wanne hinein und taucht dabei seine Füße in das Wasser unter. In dieser Stellung verharrend soll der Patient eine entsprechend der Therapie vorgegebene Dauer seine Füße in dem Wasser untergetaucht halten. Nach Ablauf dieser Dauer soll der Patient seine Füße aus dem Wasser nehmen und außerhalb der Wanne zum Beispiel zum Abtrocknen abstellen.

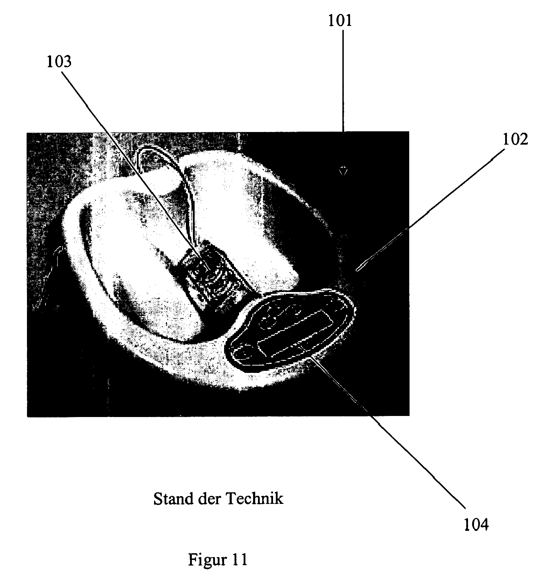

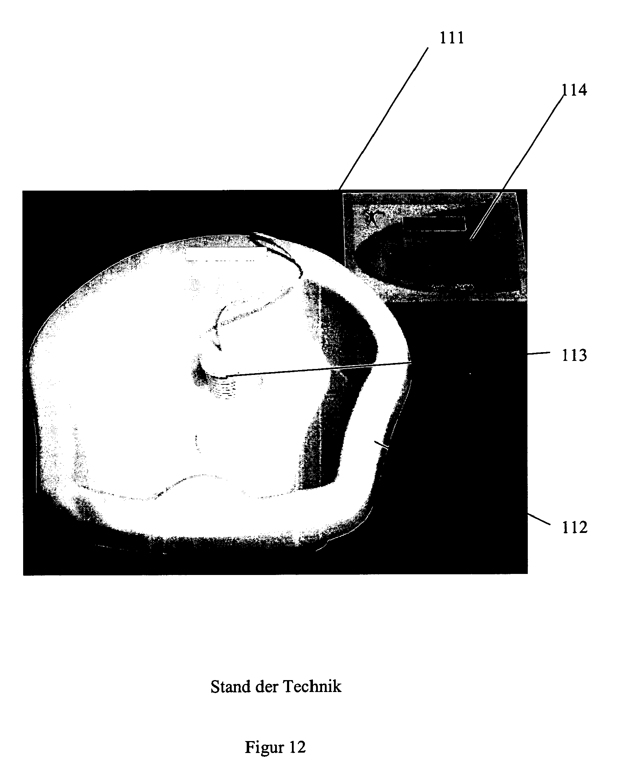

Herkömmliche Elektrolysefußbadgeräte sind in Figuren 11 und 12 gezeigt. Diese herkömmlichen Elektrolysefußbadgeräte 101, 111 sind als eine flache Wanne 102, 112 ausgebildet, auf deren Boden eine Konverterspule 103, 113 nach Figur 11 gelegt oder nach Figur 12 gestellt angeordnet ist. Bei dem herkömmlichen Elektrolysefußbadgerät 101 gemäß Figur 11 ist ein Gerät 104 zum Betreiben der Konverterspule 103 innerhalb des Elektrolysefußbadgeräts 101 integriert vorgesehen. Dadurch ist zwar dieses Elektrolysefußbadgerät 101 kompakt ausgebildet, jedoch aufgrund des beträchtlichen Eigengewichts des Geräts 104 zum Betrieb der Konverterspule 103 sehr schwer. Dadurch ist die Handhabung dieses Elektrolysefußbadgeräts 101 kompliziert.

Bei dem in Figur 12 gezeigten herkömmlichen Elektrolysefußbadgerät 111 ist ein separates Beistellgerät 114 zum Betrieb der Konverterspule 113 vorgesehen. Dadurch ist bei der Benutzung dieses Elektrolysefußbadgeräts 111 die Wanne 112 zusammen mit dem Beistellgerät 114 zu hantieren, wodurch die Handhabung kompliziert und aufwendig ist. Dadurch, dass das Beistellgerät 114 zum Betrieb des Elektrolysefußbadgeräts 111 neben der Wanne 112 abzustellen ist, besteht die Gefahr, dass beispielsweise der Patient über das Beistellgerät 114 stolpert. Dadurch ist der Betrieb dieses Elektrolysefußbadgeräts 111 unsicher.

Aufgabe der Erfindung ist es, ein Elektrolysefußbadsystem zu schaffen, dessen Handhabung einfach und dessen Betrieb sicher ist.

Das erfindungsgemäße Elektrolysefußbadsystem weist eine Fußbadwanne und einen Fußbadständer auf, auf dem die Fußbadwanne abgestützt ist. Zum Fußbaden wird einem Patienten Wasser in die Fußbadwanne gefüllt bereitgestellt. Beim Fußbaden taucht der Patient seine Füße in die Fußbadwanne ein, wobei die Füße im Wesentlichen vollständig untergetaucht sein sollen. Somit soll die in der Fußbadwanne bereitgestellte Menge Wasser entsprechend ausreichend sein, so dass, wenn die Füße in die Fußbadwanne gestellt sind, eine entsprechende Füllhöhe Wasser zur Verfügung steht, damit das Elektrolysefußbad therapeutisch entsprechend wirkungsvoll ist. Die hiermit benötigte Wassermenge kann sich bei einigen Litern belaufen, beispielsweise 5 bis 10 Liter, wodurch die entsprechend gefüllte Fußbadwanne aufgrund des Eigengewichts des Wassers ein entsprechendes Gewicht hat. Da die Fußbadwanne separat von dem Fußbadständer ausgebildet und von diesem gehalten ist, braucht die Fußbadwanne weder Betriebsgeräte noch Einrichtungen zur Erwirkung einer ausreichenden Standfestigkeit aufzuweisen, da diese Einrichtungen an den bzw. in dem Fußbadständer verwirklicht werden können. Dadurch ist die Fußbadwanne einfach und materialsparend ausgestaltet, wodurch ihr Gewicht gering ist. Somit ist die Handhabung des Elektrolysefußbadsystems beispielsweise beim Ausgießen der Fußbadwanne einfach, da hierfür lediglich die leichte und einfach handhabbare Fußbadwanne genommen zu werden braucht, wobei der Fußbadständer an Ort und Stelle verbleibt. Ferner ist das Reinigen des Elektrolysefußbadsystems einfach, insbesondere nach dessen Gebrauch, da vereinfacht die Fußbadwanne von dem Fußbadständer genommen werden zu braucht und an einem geeigneten Ort gereinigt werden kann, wobei der Fußbadständer an Ort und Stelle verbleiben kann.

Das Elektrolysefußbadsystem kann beispielsweise eine Konverterbetriebseinrichtung in einem separaten Beistellgerät aufweisen. Bevorzugt weist der Fußbadständer des Elektrolysefußbadsystems eine in ihm integrierte Konverterbetriebseinrichtung auf. Dadurch ist die Konverterbetriebseinrichtung separat von der Fußbadwanne untergebracht, wodurch die Fußbadwanne einerseits leicht und somit gut handhabbar ist und andererseits die Konverterbetriebseinrichtung einfach fern von Wasser gehalten werden kann, wodurch die Betriebssicherheit des Elektrolysefußbadsystems erhöht ist.

Die Konverterbetriebseinrichtung weist bevorzugt ein Anschlussmittel auf, mittels dessen via den Anschlussmittel ein Konverter betreibbar ist. Dadurch wird vorteilhaft erreicht, dass trotz der örtlichen Trennung der Konverterbetriebseinrichtung und des Konverters dieser von der Konverterbetriebseinrichtung betrieben werden kann.

Bevorzugt weist der Fußbadständer wenigstens einen Vorsprung auf, der in eine entsprechend komplementär am Boden der Fußbadwanne ausgebildete, in diese sich hinein streckende und dem Vorsprung zugeordnete Nase eingreift. Dadurch steht die Fußbadwanne mit dem Fußbadständer formschlüssig in Eingriff, wodurch vorteilhaft ein versehentliches seitliches Verrutschen der Fußbadwanne auf dem Fußbadständer unterbunden ist. Ferner kann die Fußbadwanne auf dem Fußbadständer ordentlich nur so platziert werden, wenn der Vorsprung in die Nase eingreift, wodurch einer Person beim Platzieren der Fußbadwanne auf den Fußbadständer geholfen wird, da der Vorsprung und die Nase als Platzierungshilfe genommen werden kann. Ferner, da die Nase nach innen in die Fußbadwanne hineinreicht, ist durch die Nase das Füllvolumen der Fußbadwanne reduziert. Dadurch ist die entsprechende Menge an Wasser reduziert, die in die Fußbadwanne gefüllt werden muss, um eine entsprechende geforderte Füllhöhe zu erreichen. Dadurch ist vorteilhaft das Gewicht der mit Wasser gefiillten Fußbadwanne vermindert, wodurch die Handhabbarkeit des Elektrolysefußbadsystems erleichtert ist.

Bevorzugt ist die Nasenseitenwand konvex geformt, so dass der Konverter unter Zusammenwirken der Nasenseitenwand mit einem entsprechend konkav ausgebildeten Gegenstück formschlüssig und somit stabil gegen Verrutschen in der Fußbadwanne für seinen Betrieb unterbringbar ist. Dadurch steht die Fußbadwanne mit ihrer Nasenseitenwand mit dem Gegenstück des Konverters formschlüssig in Eingriff, so dass der Konverter stabil in der Fußbadwanne angeordnet ist. Dadurch wird vorteilhaft verhindert, dass der Konverter beispielsweise durch Strömungen im Wasser hervorgerufen durch Bewegen der Füße in der Fußbadschale verschoben oder durch versehentliches Hinstossen verrückt wird.

Bevorzugt hat die Nasenseitenwand die Form eines Halbzylinders, dessen Längsachse im Wesentlichen senkrecht zum Fußbadwannenboden ist. Dadurch kann der Konverter von oben in die Fußbadschale mit seinem Gegenstück an der Nasenseitenwand anliegend und von dieser geführt in die Fußbadschale eingesetzt werden. Somit ist vorteilhaft das Einsetzen des Konverters in die Fußbadschale einfach zu bewerkstelligen, wobei der Konverter gegen seitliches Verrutschen stabil festgelegt ist.

Die Nasenoberseite kann beispielsweise schräg sein. Bevorzugt ist die Nasenoberseite flach, im Wesentlichen parallel zum Fußbadwannenboden ausgebildet und im Bereich der Normfüllhöhe der Fußbadwanne angeordnet. Dadurch kann vorteilhaft die Nasenoberseite als Füllmaß beim Befüllen der Fußbadwanne mit Wasser verwendet werden. Ferner steht die Nase nicht wesentlich über die Normfiillhöhe vor, so dass der Patient beispielsweise beim Hineinstellen der Füße in die Fußbadwanne oder beim Herausnehmen der Füße aus der Fußbadwanne nicht versehentlich gegen die Nase stößt.

Der Fußbadständer weist bevorzugt eine von oberhalb der Fußbadwanne zur Manipulation der Konverterbetriebseinrichtung zugängliche Konsole auf, in die die Konverterbetriebseinrichtung integriert ist. Dadurch, dass die Konverterbetriebseinrichtung in der Konsole untergebracht ist, hat der Fußbadständer eine kompakte Bauweise, wodurch seine Unterbringung wenig Platz erfordert. Ferner, da die Konsole von oben zugänglich ist, kann von einer Person die Konverterbetriebseinrichtung bequem bedient werden, insbesondere dann, wenn die Person, beispielsweise ein Patient, vor dem Elektrolysefußbadsystem sitzend und seine Füße in der Fußbadwanne badend die Konverterbetriebseinrichtung bedienen möchte.

Bevorzugt ist die Konsole an einer seitlichen Seite des Fußbadständers, unmittelbar seitlich benachbart zur Fußbadwanne angeordnet, wobei die Konsole eine Erstreckung im Wesentlichen senkrecht zum Fußbadwannenboden hat, so dass die Konsolenoberseite im Wesentlichen bündig mit der Fußbadwannenoberseite abschließt. Dadurch ist die Konsole neben der Fußbadwanne auf dem Fußbadständer angeordnet, so dass beim Herunternehmen der Fußbadwanne von dem Fußbadständer oder beim Aufsetzen der Fußbadwanne auf den Fußbadständer die Fußbadwanne seitlich von der Konsole weg bzw. seitlich zu der Konsole hin bequem bewegt werden kann, ohne mit der Fußbadwanne an die Konsole zu stoßen. Ferner, dadurch, dass die Konsolenoberseite bündig zu der Fußbadwannenoberseite verläuft, ist die Konsolenoberseite bequem von oben her zugänglich, ohne bei der Bedienung der Konverterbetriebseinrichtung mit der Fußbadwanne in Konflikt zu geraten.

Ferner weist die Konsole einen zur Fußbadwanne hin sich erstreckenden Fortsatz auf, dessen Oberseite im Wesentlichen bündig mit der Fußbadwannenoberseite abschließt. Bevorzugt ist an der Fortsatzoberseite das Anschlussmittel vorgesehen. Dadurch ragt die Konsole mit ihrem Fortsatz in den Bereich der Fußbadwanne hinein, wobei an der Fortsatzoberseite das Anschlussmittel zum Anschließen des Konverters unmittelbar benachbart zur Fußbadwannenoberseite bereitgestellt ist. Dadurch, dass das Anschlussmittel einerseits in der Nähe der Mitte der Fußbadwanne und anderseits in der Nähe der Oberseite der Fußbadwanne angeordnet ist, ist das Anschussmittel in die Nähe der in der Mitte der Fußbadwanne liegenden Position des Konverters verlegt. Somit ist das Anschlussmittel nahe des Konverters angeordnet, wodurch zum Anschließen des Konverters an das Anschlussmittel und somit an die Konverterbetriebseinheit keine Überbrückungskabel notwendig sind und der Konverter vorteilhaft direkt an das Anschlussmittel angeschlossen werden kann.

Bevorzugt ist der Fortsatz oberhalb des Vorsprungs angeordnet und greift in eine Aussparung ein, die in der Fußbadwannenoberseite sich erstreckt. Ferner liegt der Fortsatz bevorzugt senkrecht zum Wannenboden gesehen innerhalb des Vorsprungsumfangs. Dadurch erstreckt sich der Fortsatz in den von der dem Vorsprung zugeordneten Nase gebildeten Hohlraum, wodurch vorteilhaft die Fußbadwanne eine einfache Struktur aufweist, da keine zusätzliche Anpassung in der Fußbadwanne zur Unterbringung des Fortsatzes vorgenommen zu werden braucht. Ferner ist der Fortsatz via die Aussparung von oben her zugänglich, so dass der in der Fußbadwanne angeordnete Konverter vorteilhaft an das an der Fortsatzoberseite sich befindliche Anschlussmittel angeschlossen werden kann.

Der Fußbadständer weist bevorzugt eine Grundplatte auf, auf der die Fußbadwanne aufliegt. Die Grundplatte vermittelt dem Fußbadständer eine einfache und stabile Struktur, die günstig in der Herstellung ist und eine hohe Festigkeit hat.

Bevorzugt weist die Grundplatte eine Öffnung unterhalb des Tiefpunkts der Fußbadwanne auf. Dadurch kann am Tiefpunkt der Fußbadwanne ein Mittel zum Ablassen des Wassers in der Fußbadwanne vorgesehen werden, wobei durch die Öffnung der Abfluss weg von dem Elektrolysefußbadsystem vorteilhaft bewerkstelligt werden kann, insbesondere in dem durch die Öffnung zu dem Tiefpunkt der Fußbadwanne hin ein Abflussrohr verlegt werden kann.

Der Fußbadwannenboden weist bevorzugt einen lichtdurchlässigen Bereich und die Grundplatte eine lichttherapeutische Einrichtung auf, die unterhalb dem lichtdurchlässigen Bereich des Fußbadwannenbodens angeordnet ist. Dadurch ergibt sich vorteilhaft zusätzlich die Möglichkeit des Einsatzes einer Lichttherapie, wodurch die Therapiewirkung des Elektrolysefußbads verbessert werden kann.

Bevorzugt weist die lichttherapeutische Einrichtung mindestens eine in die Grundplatte eingelassene Leuchtdiode auf, die an der Oberseite der Grundplatte lichtdurchlässig abgedeckt ist. Dadurch, dass Leuchtdioden einen geringen Energieverbrauch haben, ist der Betrieb der lichttherapeutischen Einrichtung energiesparsam. Ferner, da die Leuchtdiode in die Grundplatte integriert untergebracht ist, ist diese vorteilhaft gegen äußere Einflüsse geschützt, wie beispielsweise mechanische Einwirkungen oder Feuchtigkeit.

Bevorzugt weist ferner die Grundplatte eine magnettherapeutische Einrichtung auf. Die magnettherapeutische Einrichtung kann zusammen mit der lichttherapeutischen Einrichtung betrieben oder ohne diese zur Verbesserung des Therapieeffekts des Elektrolysefußbads eingesetzt werden.

Die lichttherapeutische Einrichtung weist bevorzugt eine in die Grundplatte eingelassene Magnetfeldspule auf. Dadurch, dass die Magnetfeldspule in die Grundplatte integriert untergebracht ist, ist diese vorteilhaft unempfindlich, beispielsweise gegen mechanische äußere Einflüsse oder Feuchtigkeit.

Bevorzugt sind die lichttherapeutische Einrichtung und die magnettherapeutische Einrichtung am gleichen Ort in der Grundplatte angeordnet. Dadurch ist es ausreichend, wenn bei der Herstellung des Fußbadständers in der Grundplatte eine Aussparung vorgesehen wird, in die sowohl die lichttherapeutische Einrichtung, die magnettherapeutische Einrichtung oder beide Einrichtungen montiert werden können. Dadurch ist die Herstellung des Fußbadständers einfach und kostengünstig.

Bevorzugt sind die Fußbadwanne und/oder der Fußbadständer einstückig hergestellt. Dadurch ist die Herstellung dieser Bauteile insbesondere bei hohen Stückzahlen einfach und kostengünstig.

Die Fußbadwanne ist bevorzugt aus opalisiertem Plexiglas, dessen Boden weiß erscheint. Dadurch brauchen in der Fußbadwanne keine speziellen lichtdurchlässigen Bereiche beim Einsatz der lichttherapeutischen Einrichtung vorgesehen werden, da durch das Plexiglasmaterial das Licht der lichttherapeutischen Einrichtung für die Therapie geeignet durchscheinen kann.

Bevorzugt ist der Fußbadständer aus Kunststoff hergestellt. Insbesondere bei der Herstellung von hohen Stückzahlen ist dies vorteilhaft, da die Herstellung dieser Fußbadständer schnell und kostengünstig durchgeführt werden kann.

Bevorzugt weist die Fußbadwanne an der Oberseite einen kragenartig ausgebildeten Rand auf, so dass das Elektrolysefußbadsystem am Rand hängend gelagert in einer Mulde unterbringbar ist. Ist das Elektrolysefußbadsystem in der Mulde eingebaut, so wird das Elektrolysefußbadsystem an dem Rand abgestützt, wodurch die Abstützkraft des Elektrolysefußbadsystems oberhalb dessen Schwerpunkt an diesem angreift. Dadurch ist das Elektrolysefußbadsystem stabil gelagert, wodurch ein versehentliches Umkippen der Fußbadwanne und ein dadurch sich ergebendes Ergießen des Wassers in der Umgebung unterbunden wird.

Ferner weist die Fußbadwanne bevorzugt an der Unterseite ihres Rands ein die Fußbadwanne umfassendes Rahmenelement auf, das an dessen Innenseite den Fußbadwannenrand untergreift und in Verlängerung des Fußbadwannenrands im Wesentlichen parallel zum Fußbadwannenboden nach außen sich erstreckt, so dass via den Rahmenelement das Elektrolysefußbadsystem an der Mulde abstützbar ist.

Dadurch, dass zwischen dem Fußbadwannenrand und der Mulde das Rahmenelement vorgesehen ist, über das die Abstützkraft von der Mulde zum Fußbadwannenrand übertragen wird, liegt die Fußbadwanne mit ihrem Rand nicht direkt auf dem Rand der Mulde auf. Somit hat der Rand der Fußbadwanne keinen direkten Kontakt mit dem Rand der Mulde, wodurch ein Scheuern des Rands der Fußbadwanne am Rand der Mulde unterbunden ist, beispielsweise wenn das Elektrolysefußbadsystem mit horizontalem Spiel in der Mulde untergebracht ist und bei der Benutzung verschoben wird, beispielsweise beim Hin-und-Her-Bewegen der Füße in der Fußbadwanne. Dadurch ist ein Verschleiß an dem Rand der Fußbadschale aufgrund von Scheuern an dem Rand der Mulde unterbunden.

Bevorzugt ist das Rahmenelement aus Edelstahl hergestellt. Das Rahmenelement hat einen direkten Kontakt zum Rand der Mulde, wodurch beim Hin-und-Her-Rutschen des Elektrolysefußbadsystems in der Mulde ein Scheuern zwischen dem Rahmenelement und Rand der Mulde auftritt. Dadurch, dass Edelstahl verschleißfest ist, ist ein Verschleiß an dem Rahmenelement aufgrund von Scheuern unterbunden.

Im Folgenden wird die Erfindung anhand bevorzugter Ausführungsformen mit Bezugnahem auf die Zeichnung erläutert. In der Zeichnung zeigen:
Figur 1 eine perspektivische Ansicht einer Ausführungsform eines erfindungsgemäßen Elektrolysefußbadsystems mit einem Konverter,
Figur 2 eine Drauf- und eine Seitenansicht des Elektrolysefußbadsystems aus Figur 1 mit dem Konverter,
Figur 3 eine perspektivische Ansicht des Elektrolysefußbadsystems,
Figur 4 eine Unteransicht einer Fußbadwanne des Elektrolysefußbadsystems,
Figur 5 eine perspektivische Ansicht eines Fußbadständers des Elektrolysefußbadsystems,
Figur 6 eine Draufsicht des Fußbadständers aus Figur 5,
Figur 7 eine Seitenansicht des Fußbadständers aus Figur 5,
Figur 8 eine perspektivische Ansicht des in eine Mulde eingesetzten Elektrolysefußbadsystems,
Figur 9 die Mulde aus Figur 8 und
Figur 10 eine seitliche Schnittdarstellung des Elektrolysefußbadsystems aus Figur 8 und
Figuren 11 und 12 herkömmliche Elektrolysefußbadgeräte.

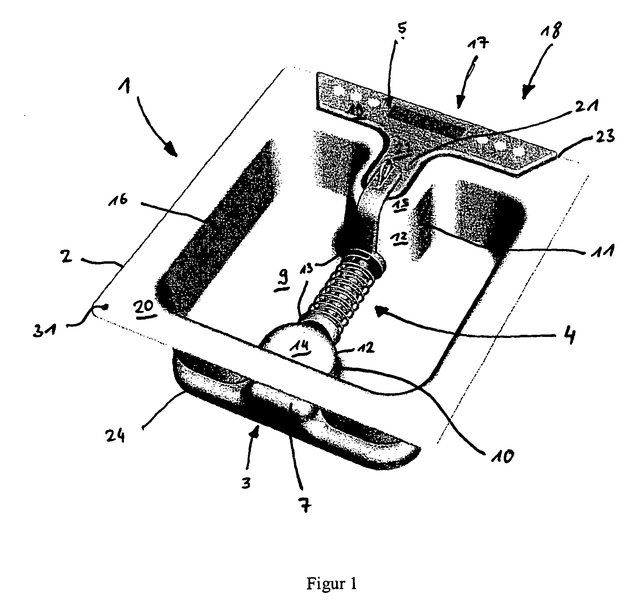

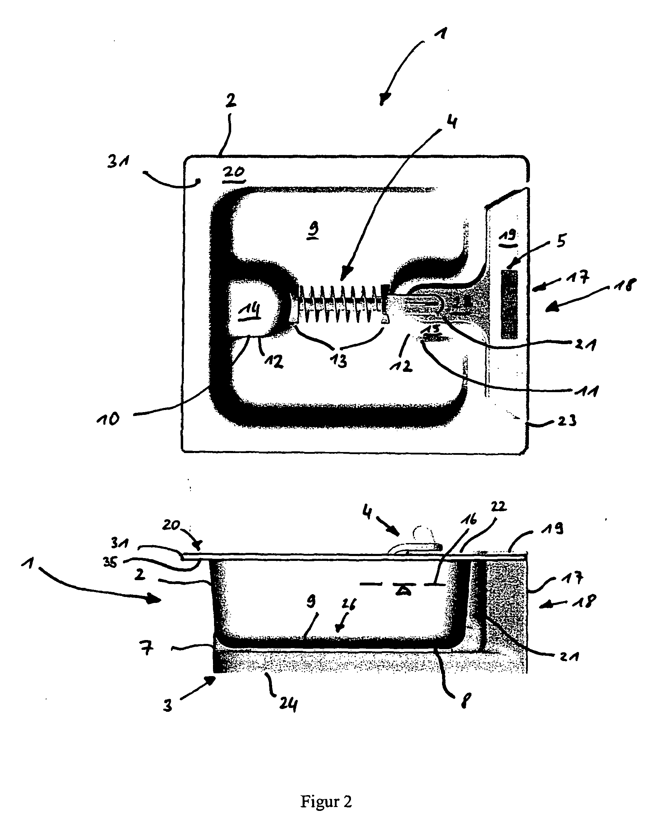

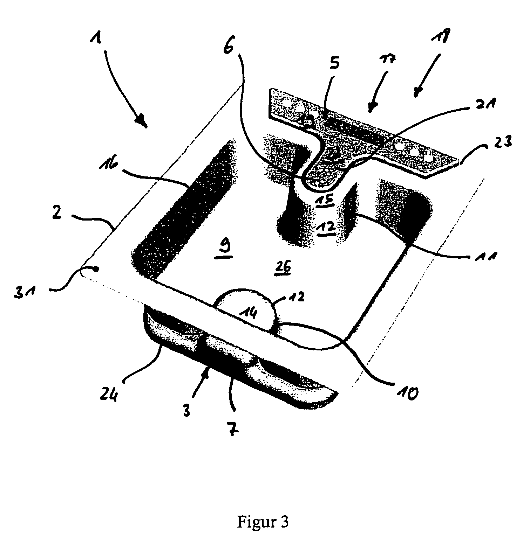

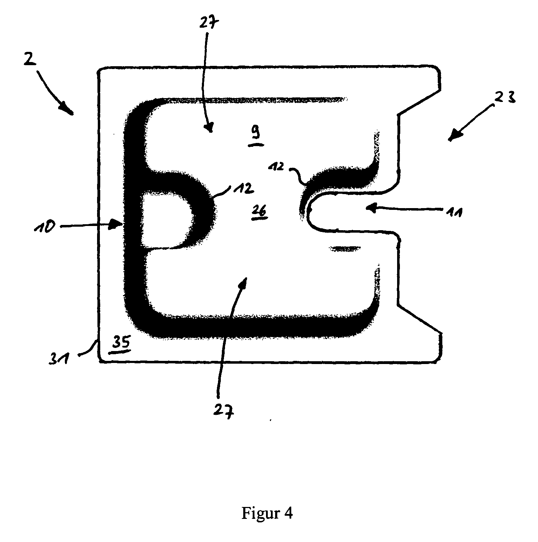

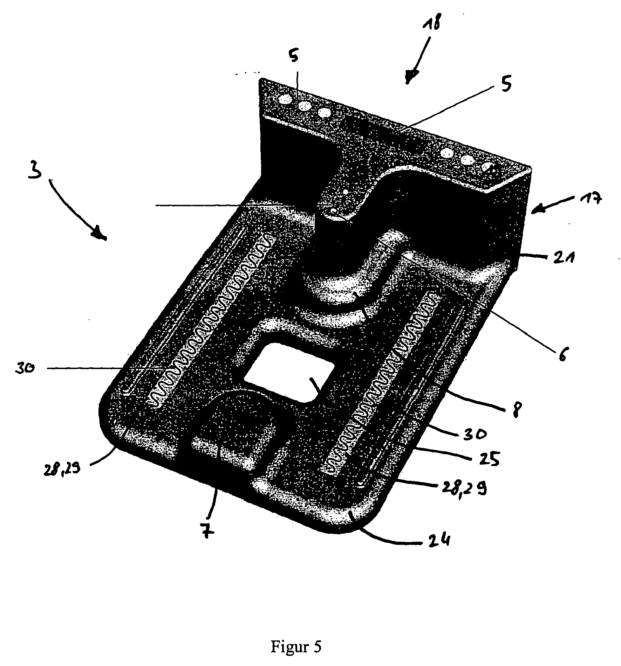

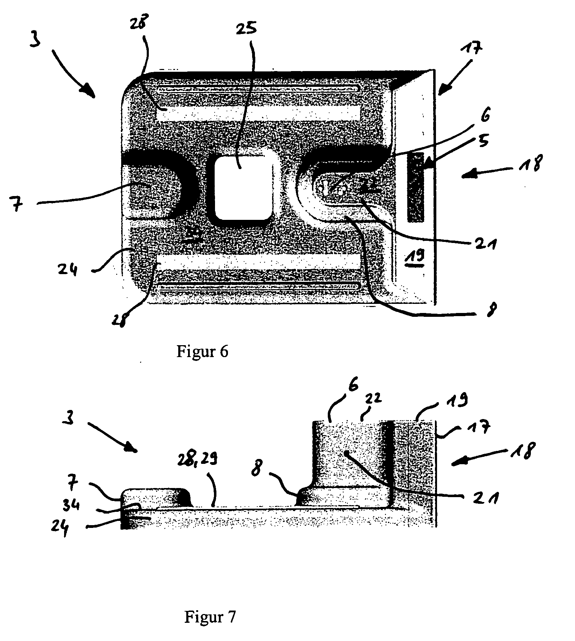

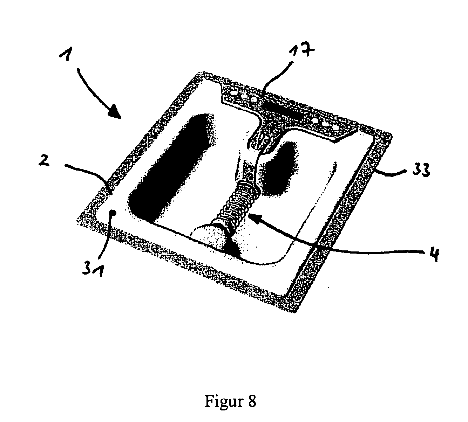

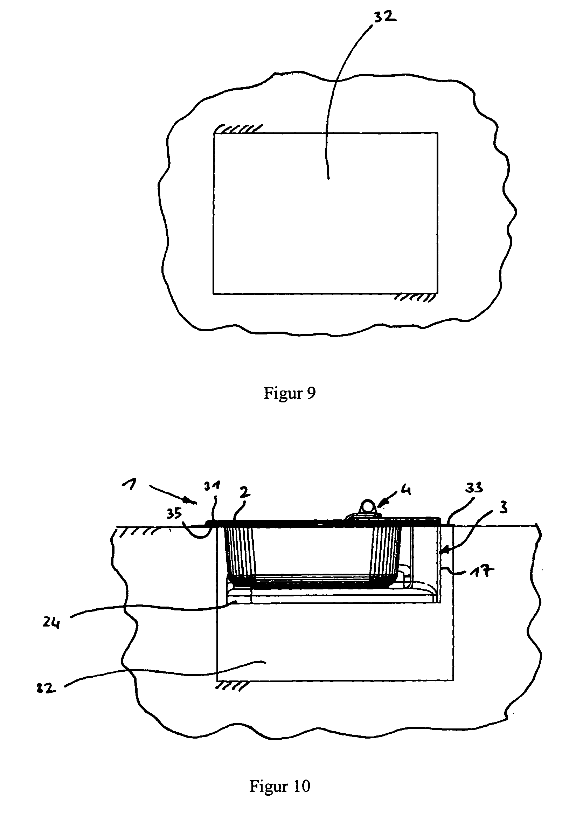

Wie es aus Figuren 1 bis 10 ersichtlich ist, weist ein erfindungsgemäßes Elektrolysefußbadsystem 1 eine Fußbadwanne 2 und einen Fußbadständer 3 mit einer Grundplatte 24 auf, auf die die Fußbadwanne 2 gestellt ist. Der Fußbadständer 3 weist eine Konverterbetriebseinrichtung 5 und ein Anschlussmittel 6 auf, wobei mit der Konverterbetriebseinrichtung 5 via den Anschlussmittel 6 ein Konverter 4 in das Elektrolysefußbadsystem 1 montiert betrieben wird.

Die Fußbadwanne 2 ist aus einem im Wesentlichen gleichdicken Flachmaterial geformt, wodurch die Fußbadwanne 2 materialsparend und somit mit minimalem Eigengewicht hergestellt ist. Dadurch, dass die Fußbadwanne 2 auf der Grundplatte 24 des Fußbadständers 3 ausreichend fest abgestützt ist, weist die Fußbadwanne 2 keine zusätzlichen Einrichtungen zur Erhöhung ihrer Standstabilität auf, die gegebenenfalls eine Erhöhung des Eigengewichts der Fußbadwanne 2 zur Folge hätten. Ferner ist die Konverterbetriebseinrichtung 5 in dem Fußbadständer 3 untergebracht und nicht in der Fußbadwanne 2, wodurch das Eigengewicht der Fußbadwanne 2 minimiert ist. Ist die Fußbadwanne 2 bis zu ihrer Normfüllhöhe 16 mit Wasser gefüllt, so beläuft sich die in der Fußbadwanne 2 befindliche Wassermenge bei 10 Liter. Hierbei ist die Fußbadwanne 2 aufgrund des Eigengewichts des Wassers entsprechend schwer, so dass aufgrund des geringen Eigengewichts der Fußbadwanne 2 diese gefüllt bei der Handhabung einfach ist.

Die Fußbadwanne 2 weist zwei Fußkammern auf, in die jeweils ein Fuß eines Patienten beim Fußbaden zu platzieren ist. Die Fußkammern werden dadurch gebildet, dass die Fußbadwanne 2 zwischen den Fußkammern eine erste Nase 10 und eine zweite Nase 11 aufweist, wobei die Nasen 10, 11 nach innen der Fußbadwanne sich erstrecken und durch Einstülpen des im Wesentlichen gleichdicken Flachmaterials der Fußbadwanne 2 hergestellt sind. Die Nasen 10, 11 sind entlang der Längsachse der Fußbadwanne 2 im Abstand zueinander angeordnet, wobei zwischen den Nasen 10, 11 der Konverter montiert ist. Die Nasen 10, 11 weisen jeweils eine Nasenseitenwand 12 auf, die die Form eines Halbzylinders hat, dessen Längsachse im Wesentlichen senkrecht zu dem Fußbadwannenboden 9 ist. Die Nasenseitenwände 12 erstrecken sich somit konvex innerhalb der Fußbadwanne 2, so dass jeweils ein entsprechend gegenförmig mit halbzylindrischer Oberfläche ausgestattetes Gegenstück 13 des Konverters 4 an den Nasenseitenwänden 12 anliegt. Dadurch kann der Konverter 4 leicht von oben mittels der Gegenstücke 13 an den Nasenseitenwänden 12 entlang geführt eingesetzt werden, wobei der Konverter 4 aufgrund des Formschlusses zwischen den Nasenseitenwänden 12 und den Gegenstücken 13 gegen seitliches Verrutschen parallel zu dem Fußbadwannenboden 9 gesichert ist.

Die erste Nase 10 weist eine flache, parallel zu dem Fußbadwannenboden 9 ausgebildete erste Nasenoberseite 14 auf. Die erste Nasenoberseite 14 ist in dem Bereich der Normfüllhöhe 16 der Fußbadwanne 2 angesiedelt, so dass beim Befüllen der Fußbadwanne 2 die erste Nasenoberseite 14 als Füllmarkierung genommen werden kann.

Da die Nasen 10, 11 in die Fußbadwanne 2 nach innen sich erstrecken, ist durch die Ausdehnung der Nasen 10, 11 das Füllvolumen der Fußbadwanne 2 reduziert, wodurch das Gewicht der gefüllten Fußbadwanne 2 reduziert ist.

Die Grundplatte 24 des Fußbadständers 3 weist einen ersten Vorsprung 7 und einen zweiten Vorsprung 8 auf, die jeweils komplementär zu der ersten Nase 10 und der zweiten Nase 11 ausgebildet sind, so dass der erste Vorsprung 7 in den von der ersten Nase 10 gebildeten Hohlraum und der zweite Vorsprung 8 in den von der zweiten Nase 11 gebildeten Hohlraum von unten her sich erstreckt. Dadurch ist die Fußbadwanne 2 durch das formschlüssige Zusammenwirken der Vorsprünge 7, 8 mit den Nasen 10, 11 gegen seitliches Verrutschen gesichert. Ferner kann beim Hinstellen der Fußbadwanne 2 auf die Grundplatte 24 das Eingreifen der Vorsprünge 7, 8 in die Nasen 10, 11 als Markierung für die richtige Positionierung der Fußbadwanne 2 auf der Grundplatte 24 genommen werden.

Zwischen der ersten Nase 10 und der zweiten Nase 11 ist am Wannenboden 9 ein Tiefpunkt ausgebildet. Unter dem Tiefpunkt des Wannenbodens 9 ist in der Grundplatte 24 eine Öffnung 25 vorgesehen, so dass die Fußbadwanne 2 an ihrem Tiefpunkt durch eine Abflusseinrichtung ausbaubar ist, wobei die Abflusseinrichtung durch die Öffnung 25 geführt werden kann.

Auf der Grundplatte 24 des Fußbadständers 3 ist eine Konsole 17 vorgesehen, in der die Konverterbetriebseinrichtung 5 untergebracht ist. Der Fußbadständer 3 hat somit eine kompakte Bauweise, wodurch seine Unterbringung vor Ort wenig Platz erfordert.

Die Konsole 17 erstreckt sich senkrecht nach oben von der Grundplattenoberseite 34 bündig bis zur Oberseite der Fußbadwanne 2, wobei in der Fußbadwanne 2 eine Aussparung 23 vorgesehen ist, so dass die Konsole 17 von oben durch die Aussparung 23 zugänglich ist. In der Konsolenoberseite 19 der Konsole 17 ist die Konverterbetriebseinrichtung 5 so zugänglich angeordnet, dass die Betriebseinrichtung 5 von oben her bequem bedient werden kann.

Die Konsole 17 ist an derjenigen seitlichen Seite 18 des Fußbadständers 3 angeordnet, die bei gewöhnlicher Benutzung des Elektrolysefußbadsystems 1 den Zehen der in der Fußbadwanne 2 platzierten Füße des Patienten benachbart angeordnet ist. Somit sitzt für gewöhnlich der Patient vor dem Elektrolysefußbadsystem 1 an der Seite, die der Konsole 17 abgewandt ist. Dadurch ist die Konsolenoberseite 19 mit der von oben her manipulierbaren Konverterbetriebseinrichtung 5 von dem Patienten gut einsehbar, da der Patient seine Oberschenkel beim Betrachten der Konsolenoberseite 19 nicht im Blickfeld hat. Ferner ist die Fußbadwanne 2 von drei Seiten her leicht zugänglich, nämlich von links, von rechts und von vorne, ohne dass die Konsole 17 dem Patienten im Wege steht.

Die Konsole 17 weist einen Fortsatz 21 auf, der in Richtung zur Fußbadwanne 2 hin horizontal sich erstreckt und in Verlängerung nach oben zu dem zweiten Vorsprung 8 ausgebildet ist. Die Fortsatzoberseite 22 ist in Verlängerung zur Konsolenoberseite 19 ausgebildet, so dass die Fortsatzoberseite 22 bündig zur Fußbadwannenoberseite 20 verläuft. An der Fortsatzoberseite 22 ist das Anschlussmittel 6 für den Anschluss des Konverters 4 an die Konverterbetriebseinrichtung 5 vorgesehen. Die Aussparung 23 an der Fußbadwannenoberseite 20 ist so ausgebildet, dass sie den Fortsatz 21 so umgreift, dass das Anschlussmittel 6 von oben her zugänglich ist.

Die zweite Nase 11 ist derart ausgebildet, dass sowohl der zweite Vorsprung 8 als auch der Fortsatz 21 in den von der zweiten Nase 11 gebildeten Hohlraum an der Außenseite der Fußbadwanne 2 eingreifen. Der Fortsatz 21 weist einen horizontalen Querschnitt auf, der kleiner ist als der des zweiten Vorsprungs 8, so dass die Oberseite 15 der zweiten Nase 11 unter Ausbildung eines Kragens um den Fortsatz 21 herum zur Fortsatzoberseite 22 hin sich erstreckt. Dadurch ist die Fortsatzoberseite 22 und somit das Anschlussmittel 6 vor Spritzwasser geschützt ist.

Ferner, da das Anschlussmittel 6 innerhalb der zweiten Nase 11 auf der Höhe der Fußbadwannenoberseite 20 angeordnet ist, ist das Anschlussmittel 6 in die Nähe der Position des Konverters 4 gebracht, wodurch der Überbrückungsabstand zwischen dem Anschlussmittel 6 und dem Konverter 4 gering ist. Dadurch kann der Konverter 4 direkt an das Anschlussmittel 6 angeschlossen werden, ohne dass Verbindungskabel oder dergleichen notwendig sind.

Die Fußbadwanne 2 weist an ihrer Oberseite 20 einen Fußbadwannenrand 31 auf, der kragenartig nach außen horizontal sich erstreckend ausgebildet ist. An der Unterseite 35 des Rands 31 weist die Fußbadwanne 2 ein Rahmenelement 33 auf, das das Elektrolysefußbadsystem 1 umgreift und an dessen Innenseite den Fußbadwannenrand 31 untergreift. Das Rahmenelement 33 ist ein flacher Metallrahmen aus Edelstahl, der in Verlängerung zu dem Fußbadwannenrand 31 angeordnet ist. Das Elektrolysefußbadsystem 1 kann in eine Mulde 32 eingesetzt werden, wobei das Elektrolysefußbadsystem 1 an der Mulde 32 von dem Rahmenelement 33 abgestützt ist. Dadurch wird unterbunden, dass das Elektrolysefußbadsystem 1 direkt mit ihrem Fußbadwannenrand 31 auf dem Rand der Mulde aufliegt, wodurch ein Scheuern des Rands 31 an dem Rand der Mulde unterbunden ist, beispielsweise wenn das Elektrolysefußbadsystem 1 horizontal verrutschbar in der Mulde 32 untergebracht ist und bei der Benutzung beim Hin-und-Her-Bewegen der Füße verschoben wird.

Stellt der Patient seine Füße in die Fußbadwanne 2, so ergeben sich an dem Fußbadwannenboden 9 Bereiche 27, die für gewöhnlich von den Fußsohlen berührt werden. Diese Bereiche 27 sind lichtdurchlässig ausgebildet. Unterhalb diesen Bereichen 27 ist in der Grundplattenoberseite 34 der Grundplatte 24 eine lichttherapeutische Einrichtung 28 in Form eines Leuchtdiodenbands eingelassen, wobei das Leuchtdiodenband an der Oberseite 34 der Grundplatte 24 lichtdurchlässig abgedeckt ist. In den Leuchtdiodenbändern integriert ist eine magnettherapeutische Einrichtung 29 in Form einer lang gestreckten Magnetfeldspule 30 vorgesehen. Da die Leuchtdiodenbänder und die Magnetfeldspulen 30 in die Grundplatte 24 integriert untergebracht sind, sind diese geschützt gegen äußere Einflüsse, wie beispielsweise mechanische Einwirkungen oder Feuchtigkeit. Ferner, da die lichttherapeutische Einrichtung 28 Leuchtdioden aufweist, ist der Betrieb der lichttherapeutischen Einrichtung 28 energiesparsam, weil die Leuchtdiodenbänder einen geringen Energieverbrauch haben.

Dadurch, dass in dem Elektrolysefußbadsystem 1 sowohl die lichttherapeutische Einrichtung 28 als auch die magnettherapeutische Einrichtung 29 vorgesehen sind, können diese in Kombination oder einzeln zusammen mit dem Elektrolysefußbad zur Verbesserung des Therapieeffekts des Elektrolysefußbads betrieben werden. Sowohl die lichttherapeutische Einrichtung 28 als auch die magnettherapeutische Einrichtung werden von der Konverterbetriebseinrichtung 5 betrieben, so dass zum Betreiben der lichttherapeutischen Einrichtung 28 und der magnettherapeutischen Einrichtung 29 zusätzliche Betriebsgeräte nicht vorgesehen zu werden brauchen.

Da die Magnetfeldspule 30 in dem Leuchtdiodenband der lichttherapeutischen Einrichtung 28 integriert ist, sind beide Einrichtungen 28, 29 am gleichen Ort in der Grundplatte 24 angeordnet. Dadurch ist es ausreichend, wenn bei der Herstellung des Fußbadständers 3 in der Grundplatte 24 eine Aussparung vorgesehen wird, in die sowohl die lichttherapeutische Einrichtung 29 als auch beiden Einrichtungen 28, 29 montiert werden können. Dadurch ist die Herstellung des Fußbadständers 3 einfach und kostengünstig.

### Bezugszeichenliste

- 1: Elektrolysefußbadsystem
- 2: Fußbadwanne
- 3: Fußbadständer
- 4: Konverter
- 5: Konverterbetriebseinrichtung
- 6: Anschlussmittel
- 7: erster Vorsprung
- 8: zweiter Vorsprung
- 9: Fußbadwannenboden
- 10: erste Nase
- 11: zweite Nase
- 12: Nasenseitenwand
- 13: Gegenstück des Konverters
- 14: erste Nasenoberseite
- 15: zweite Nasenoberseite
- 16: Normfüllhöhe
- 17: Konsole
- 18: Seitliche Seite des Fußbadständers
- 19: Konsolenoberseite
- 20: Fußbadwannenoberseite
- 21: Fortsatz
- 22: Fortsatzoberseite
- 23: Aussparung
- 24: Grundplatte
- 25: Öffnung
- 26: Tiefpunkt der Fußbadwanne
- 27: Lichtdurchlässiger Bereich des Fußbadswannenbodens
- 28: Lichttherapeutische Einrichtung
- 29: Magnettherapeutische Einrichtung
- 30: Magnetfeldspule
- 31: Fußbadwannenrand

- 32: Mulde
- 33: Rahmenelement
- 34: Grundplattenoberseite
- 35: Unterseite des Fußbadwannenrands

## Patentansprüche

1. Elektrolysefußbadsystem mit einer Fußbadwanne (2) und einem Fußbadständer (3), auf dem die Fußbadwanne (2) abgestützt ist.

2. Elektrolysefußbadsystem gemäß Anspruch 1, wobei der Fußbadständer (3) eine in ihm integrierte Konverterbetriebseinrichtung (5) mit einem Anschlussmittel (6) aufweist, mittels dessen via den Anschlussmittel (6) ein Konverter (4) betreibbar ist.

3. Elektrolysefußbadsystem gemäß Anspruch 1 oder 2, wobei der Fußbadständer (3) wenigstens einen Vorsprung (7, 8) aufweist, der in eine entsprechend komplementär am Boden (9) der Fußbadwanne (2) ausgebildete, in diese hinein sich erstreckende und dem Vorsprung (7, 8) zugeordnete Nase (10, 11) eingreift.

4. Elektrolysefußbadsystem gemäß Anspruch 3, wobei die Nasenseitenwand (12) konvex geformt ist, so dass der Konverter (4) unter Zusammenwirken der Nasenseitenwand (12) mit einem entsprechend konkav ausgebildeten Gegenstück (13) des Konverters (4) formschlüssig und somit stabil gegen Verrutschen in der Fußbadwanne (2) für seinen Betrieb unterbringbar ist.

5. Elektrolysefußbadsystem gemäß Anspruch 4, wobei zwei Vorsprünge (7, 8) an dem Fußbadständer (3) und entsprechend zwei Nasen (10, 11) in der Fußbadwanne (2) vorgesehen sind, die mit ihren konvex geformten Nasenseitenwänden (12) sich gegenüberliegend und im Abstand zueinander angeordnet sind, so dass zwischen den Nasenseitenwänden (12) der Konverter (4) unterbringbar ist.

6. Elektrolysefußbadsystem gemäß Anspruch 4 oder 5, wobei die Nasenseitenwand (12) die Form eines Halbzylinders hat, dessen Längsachse im Wesentlichen senkrecht zum Fußbadwannenboden (9) ist.

7. Elektrolysefußbadsystem gemäß Anspruch 6, wobei die Nasenoberseite (14, 15) flach, im Wesentlichen parallel zum Fußbadwannenboden (9) ausgebildet und im Bereich der Normfüllhöhe (16) der Fußbadwanne (2) angeordnet ist.

8. Elektrolysefußbadsystem gemäß Anspruch 6 oder 7, wobei der Fußbadständer (3) eine von oberhalb der Fußbadwanne (2) zur Manipulation der Konverterbetriebseinrichtung (5) zugängliche Konsole (17) aufweist, in die die Konverterbetriebseinrichtung (5) integriert ist.

9. Elektrolysefußbadsystem gemäß Anspruch 8, wobei an einer seitlichen Seite des Fußbadständers (3), unmittelbar seitlich benachbart zur Fußbadwanne (2) die Konsole (17) angeordnet ist, die eine Erstreckung im Wesentlichen senkrecht zum Fußbadwannenboden (9) hat, so dass die Konsolenoberseite (19) im Wesentlichen bündig mit der Fußbadwannenoberseite (20) abschließt.

10. Elektrolysefußbadsystem gemäß Anspruch 9, wobei die Konsole (17) einen zur Fußbadwanne (2) hin sich erstreckenden Fortsatz (21) aufweist, dessen Oberseite (22) im Wesentlichen bündig mit der Fußbadwannenoberseite (20) abschließt.

11. Elektrolysefußbadsystem gemäß Anspruch 10, wobei an der Fortsatzoberseite (22) das Anschlussmittel (6) vorgesehen ist.

12. Elektrolysefußbadsystem gemäß Anspruch 10 oder 11, wobei der Fortsatz (21) oberhalb des Vorsprungs (7, 8) angeordnet ist und in eine Aussparung (22) eingreift, die in der Fußbadwannenoberseite (20) sich erstreckt.

13. Elektrolysefußbadsystem gemäß Anspruch 12, wobei der Fortsatz (21) senkrecht zum Fußbadwannenboden (9) gesehen innerhalb des Vorsprungsumfangs liegt.

14. Elektrolysefußbadsystem gemäß einem der Anspruch 1 bis 13, wobei der Fußbadständer (3) eine Grundplatte (24) aufweist, auf der die Fußbadwanne (2) aufliegt.

15. Elektrolysefußbadsystem gemäß Anspruch 14, wobei die Grundplatte (24) eine Öffnung (25) unterhalb eines Tiefpunkts (26) der Fußbadwanne (2) aufweist.

16. Elektrolysefußbadsystem gemäß Anspruch 14 oder 15, wobei der Fußbadwannenboden (9) einen lichtdurchlässigen Bereich (27) und die Grundplatte (24) eine lichttherapeutische Einrichtung (28) aufweist, die unterhalb im lichtdurchlässigen Bereich (27) des Fußbadwannenbodens (9) angeordnet ist.

17. Elektrolysefußbadsystem gemäß Anspruch 16, wobei die lichttherapeutische Einrichtung (28) eine in die Grundplatte (24) eingelassene Leuchtdiode aufweist, die an der Oberseite der Grundplatte (24) lichtdurchlässig abgedeckt ist.

18. Elektrolysefußbadsystem gemäß einem der Ansprüche 14 bis 17, wobei die Grundplatte (24) eine magnettherapeutische Einrichtung (29) aufweist.

19. Elektrolysefußbadsystem gemäß Anspruch 18, wobei die magnettherapeutische Einrichtung (29) eine in die Grundplatte (24) eingelassene Magnetfeldspule (30) aufweist.

20. Elektrolysefußbadsystem gemäß Anspruch 18 oder 19, wobei die lichttherapeutische Einrichtung (28) und die magnettherapeutische Einrichtung (29) am gleichen Ort in der Grundplatte (24) angeordnet sind.

21. Elektrolysefußbadsystem gemäß einem der Ansprüche 1 bis 20, wobei die Fußbadwanne (2) einstückig hergestellt ist.

22. Elektrolysefußbadsystem gemäß Anspruch 21, wobei die Fußbadwanne (2) aus opalisiertem Plexiglas ist, dessen Boden (9) weiß erscheint.

23. Elektrolysefußbadsystem gemäß einem der Ansprüche 1 bis 22, wobei der Fußbadständer (3) einstückig hergestellt ist.

24. Elektrolysefußbadsystem gemäß Anspruch 23, wobei der Fußbadständer (3) aus Kunststoff hergestellt ist.

25. Elektrolysefußbadsystem gemäß einem der Ansprüche 1 bis 24, wobei die Fußbadwanne (2) an ihrer Oberseite (22) einen kragenartig ausgebildeten Rand (31) aufweist, so dass das Elektrolysefußbadsystem (1) am Rand (31) hängend gehalten in einer Mulde (32) unterbringbar ist.

26. Elektrolysefußbadsystem gemäß Anspruch 25, wobei die Fußbadwanne (2) an der Unterseite (36) ihres Rands ein die Fußbadwanne (2) umfassendes Rahmenelement (33) aufweist, das an dessen Innenseite den Fußbadwannenrand (31) untergreift und in Verlängerung des Fußbadwannenrands (31) im Wesentlichen parallel zum Fußbadwannenboden (9) nach außen sich erstreckt, so dass via den Rahmenelement (33) das Elektrolysefußbadsystem (1) an der Mulde (32) abstützbar ist.

27. Elektrolysefußbadsystem gemäß Anspruch 26, wobei das Rahmenelement (33) aus Edelstahl hergestellt ist.
